# EUROPÄISCHE PATENTSCHRIFT

(11) **EP 2 303 847 B1**
(45) Veröffentlichungstag und Bekanntmachung des Hinweises auf die Patenterteilung: **05.09.2018**
(21) Anmeldenummer: 09777236.2
(22) Anmeldetag: 16.07.2009
(51) Int. Cl.: C07D 233/26, C07D 239/06, C07D 243/04

(54) **SYNTHESE VON ZYKLISCHEN AMIDINEN**
SYNTHESIS OF CYCLIC AMIDINES
SYNTHÈSE D AMIDINES CYCLIQUES

(30) Priorität: 16.07.2008 DE 102008033448
(43) Veröffentlichungstag der Anmeldung: 06.04.2011
(73) Patentinhaber: bitop AG, 58453 Witten (DE)
(72) Erfinder: LENTZEN, Georg, 46483 Wesel (DE); NEUHAUS, Thorsten, 44789 Bochum (DE)
(74) Vertreter: Schöneborn, Holger
(86) Internationale Anmeldenummer: PCT/EP2009/005175
(87) Internationale Veröffentlichungsnummer: WO 2010/006792

(56) Entgegenhaltungen:
- DE-A1- 4 342 560
- JP-A- 3 031 265
- G.J. SHAW ET AL.: "2,4-diamino-3-methylbutanoic acid, a novel amino acid in root nodule hydrolysates from lotus tenius" PHYTOCHEMISTRY, Bd. 20, Nr. 8, 1981, XP002552435
- STARMANS W A J ET AL: "Enzymatic resolution of methyl N-alkyl-azetidine-2-carboxylates by Candida antarctica lipase-mediated ammoniolysis" TETRAHEDRON ASYMMETRY, PERGAMON PRESS LTD, OXFORD, GB, Bd. 9, Nr. 3, 13. Februar 1998 (1998-02-13), Seiten 429-435, XP004131189 ISSN: 0957-4166

## Beschreibung

Die Erfindung betrifft ein Verfahren zur Herstellung von zyklischen Amidinen. Des Weiteren betrifft die Erfindung verschiedene zyklische Amidine selbst. Insbesondere kann es sich bei den zyklischen Amidinen um Ectoin, Ectoinderivate und Ectoinanaloga handeln.

Osmolyte bzw. kompatible Solute aus extremophilen Mikroorganismen bilden eine bekannte Gruppe niedermolekularer Schutzstoffe. Extremophile sind sehr außergewöhnliche Mikroorganismen, da sie optimal bzw. bei hohen Salzkonzentrationen (bis 200 g NaCl/l) und hohen Temperaturen (60 bis 110°C) wachsen, die bei mesophilen (normalen) Organismen zu massiven Schädigungen zellulärer Strukturen führen würden. In den letzten Jahren wurde daher ein großer Forschungsaufwand betrieben, um die biochemischen Komponenten zu identifizieren, die zu der bemerkenswerten Stabilisierung der Zellstrukturen führen. Obwohl viele Enzyme aus hyperthermophilen Mikroorganismen auch unter hohen Temperaturen stabil sind, gilt dies nicht generell für die zellulären Strukturen thermo- und hyperthermophiler Organismen. Zu der hohen Temperaturstabilität von Zellstrukturen tragen in erheblichem Maße niedermolekulare organische Substanzen (kompatible Solute, Osmolyte) im intrazellulären Milieu bei. Verschiedene neuartige Osmolyte konnten in den letzten Jahren in extremophilen Mikroorganismen erstmals identifiziert werden. In einigen Fällen konnte der Beitrag dieser Verbindungen zum Schutz zellulärer Strukturen - vor allem Enzymen - gegenüber Hitze und Trockenheit bereits gezeigt werden (K. Lippert, E. A. Galinski, Appl. Microbiol. Biotech. 1994, 37, 61-65; P. Louis, H. G. Trüper, E. A. Galinski, Appl. Microbiol. Biotech. 1994, 41, 684-688; Ramos et al., Appl. Environm. Microbiol. 1997, 63, 4020-4025; Da Costa, Santos, Galinski, Adv. in Biochemical Engineering Biotechnology, 61, 117-153).

Für eine Reihe von kompatiblen Soluten haben sich sinnvolle Anwendungsmöglichkeiten im medizinischen, kosmetischen und biologischen Bereich ergeben. Zu den wichtigsten kompatiblen Soluten zählt dabei das Ectoin (1,4,5,6-Tetrahydro-2-methyl-pyrimidin-4-carbonsäure) bzw. seine Derivate. So wird beispielsweise in der EP 0 887 418 A2 die Verwendung von Ectoin und Hydroxyectoin zur Behandlung von Hauterkrankungen oder als wirksamer Zusatz zur Kryoprotektion von biologischen Wirkstoffen und Zellen beschrieben. Die DE 10 2006 056 766 A1 zeigt die Verwendung von Ectoinen zur Behandlung des Vascular Leak Syndroms (VLS). Weitere Beispiele sind die Stabilisierung von Vakzinen (DE 100 65 986 A1), die Behandlung von auf Schwebstaubeinwirkung beruhenden Lungenkrankheiten und kardiovaskulären Erkrankungen (DE 103 30 768 A1) oder die dermatologische Verwendung zur Behandlung von Neurodermitis (DE 103 30 243 A1).

Kompatible Solute wie Ectoine und Hydroxyectoine lassen sich unter Hochsalzbedingungen sehr gut in Bakterien wie *Halomonas elongata* oder *Marinococcus halophilus* anreichern und anschließend aus der Trockenmasse isolieren. Eine Möglichkeit besteht dabei im sogenannten "Bakterienmelken", bei dem die Salinität des Mediums gesenkt wird, nachdem die Zellen bei hoher Salinität große Mengen an Ectoin produziert haben. Die Senkung der Salinität führt dazu, dass die Bakterien das Ectoin ins Medium ausschleusen, aus welchem es isoliert und chromatographisch aufgereinigt werden kann. Die Zellen selbst bleiben dabei intakt und können mehrfach "gemolken" werden (T. Sauer, E. A. Galinski, Biotech. Bioeng., 1998, 57, 306-313). Der Vorteil eines solchen Verfahrens gegenüber einer rein chemischen Synthese ist insbesondere die Stereoselektivität der Biosynthese. Entsprechend wird lediglich das L-Ectoin in enantiomerenreiner Form gewonnen. Ein Nachteil der Biosynthese ist jedoch, dass sie auf Substanzen beschränkt ist, die in den Bakterien selbst als Endprodukte akkumuliert werden, d. h. eine Derivatisierung ist allenfalls in beschränktem Maße ausgehend vom gewonnenen Ectoin möglich.

Grundsätzlich bekannt ist auch die chemische Synthese von Ectoin nach Koichi et al. (JP-A-03031265). Bei diesem Verfahren wird Orthoessigsäuretrimethylester mit 2,4-Diaminobuttersäure umgesetzt. Bei der Reaktion wird zunächst die 2,4-Diaminobuttersäure acetyliert, wobei anschließend bei erhöhter Temperatur ein kondensierender Ringschluss zum gewünschten Produkt erfolgt. DE 4342560 offenbart ein Verfahren zur Herstellung von Ectoinderivaten. Neben Ectoin und seinen Derivaten sind auch entsprechende 5- und 7-Ringanaloga bekannt. Das entsprechende 7-Ringanalogon wird auch Homoectoin (4,5,6,7-Tetrahydro-2-methyl-1H-[1,3]-diazepin-4-carbonsäure), das entsprechende 5-Ringanalogon DHMICA (4,5-Dihydro-2-methyl-imidazol-4-carbonsäure) genannt. Homoectoin wird entsprechend der Synthese nach Koichi durch Ringschluss zwischen Orthoessigsäuretrimethylester und Ornithin, DHMICA durch Ringschluss zwischen Orthoessigsäuretrimethylester und 2,3-Diaminopropionsäure erhalten. Homoectoin und DHMICA haben in ersten Untersuchungen vielversprechende Eigenschaften gezeigt.

Nach wie vor existiert jedoch kein allgemeiner Syntheseweg, um verschiedene zyklische Amidine in Form eines Enantiomers zu erzeugen, die in ihrem Grundgerüst Ectoin, Homoectoin oder DHMICA entsprechen. Es stellt sich daher die Aufgabe, ein derartiges Verfahren zur Verfügung zu stellen.

Diese Aufgabe wird erfindungsgemäß gelöst durch Verfahren gemäß den Ansprüchen 1 bis 3.

Die erfindungsgemäße Synthesestrategie geht aus von einem Lacton der allgemeinen Formel II, das durch eine Bromierung zur Dibromverbindung III umgesetzt wird. Die Bromierung erfolgt in Gegenwart von Brom und PBr₃. Anstelle des direkten Einsatzes von PBr₃ ist auch der Einsatz von Phosphor möglich.

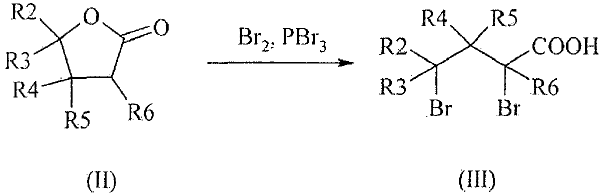

Das durch die Bromierung erhaltene 2,4-Dibrombutyrat III muss für die Folgereaktionen verestert werden, wodurch man zum entsprechenden Ester IV des 2,4-Dibrombutyrats gelangt. Die Veresterung ist zum einen im Hinblick auf die Folgeumsetzungen von Bedeutung, darüber hinaus sollte eine Veresterung auch deshalb möglichst sofort durchgeführt werden, um die Bildung des entsprechenden α-Brom-γ-butyrolactons aus der Dibromverbindung III unter HBr-Abspaltung zu verhindern. Die Veresterung erfolgt bevorzugt durch Umsetzung mit Methanol oder Ethanol in saurem Milieu. Dabei kann die Veresterung insbesondere unmittelbar im Anschluss an die Bromierung, d. h. ohne zwischenzeitliche Isolierung der Verbindung III erfolgen, beispielsweise durch Zugabe von absolutem Methanol oder Ethanol unter Hindurchleiten von gasförmigem Chlorwasserstoff HCl.

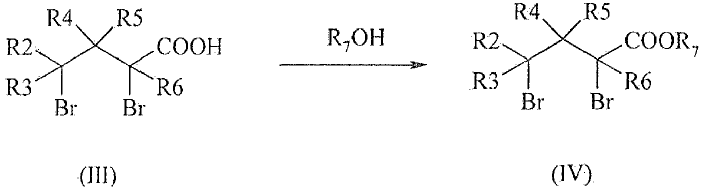

Durch gezielte Variation der Substituenten R2, R3, R4, R5 und R6 am Butyrolacton II können entsprechend substituierte Ectoinderivate hergestellt werden. Bei den Resten R2, R3, R4, R5, R6 und R7 kann es sich um H oder Alkyl-, Cycloalkyl-, Arylalkyl-, Alkoxyalkyl-, Alkylthioalkyl-, Aryloxyalkyl- oder Arylthioalkylreste handeln. Es steht somit eine große Bandbreite an Variationsmöglichkeiten zur Verfügung. Bevorzugt allerdings handelt es sich bei den Resten R2, R3, R4, R5 und R6 um H oder C₁- bis C₆-Alkyl. Häufig wird dabei eine Substitution lediglich an einer der Positionen R2, R3, R4, R5 und R6 vorliegen, während die jeweils anderen Reste ein H-Atom darstellen.

Nach Herstellung der veresterten Dibromverbindung IV wird eine Aminierung zur Diaminoverbindung V durchgeführt. Die Substitution des Broms durch Aminogruppen kann entweder durch direkte Umsetzung mit Ammoniak oder durch nucleophile Substitution mit einem Azid gefolgt von einer anschließenden Hydrierung erfolgen. Die direkte Umsetzung mit Ammoniak kann in konzentrierter wässriger Ammoniaklösung bei erhöhter Temperatur von ca. 50°C über einen längeren Zeitraum von ca. 20 bis 30 Stunden erfolgen, durch Umsetzung mit Ammoniak in einem Autoklaven oder in flüssigem Ammoniak. In letzterem Fall beispielsweise wird Ammoniak in einem Kolben bei ca. -40°C einkondensiert und ggf. mit wasserfreiem Äther verdünnt. Die Dibromverbindung IV wird langsam hinzugefügt. Die Reaktion findet über einen Zeitraum von ca. 2 Stunden statt.

Alternativ kann auch eine nucleophile Substitution der Bromatome mit einem Azid durchgeführt werden, wobei bevorzugt Natriumazid NaN₃ eingesetzt wird. Anschließend erfolgt eine Hydrierung unter Standardbedingungen über einem geeigneten Katalysator, beispielsweise Palladium/Aktivkohle Pd/C oder Platin.

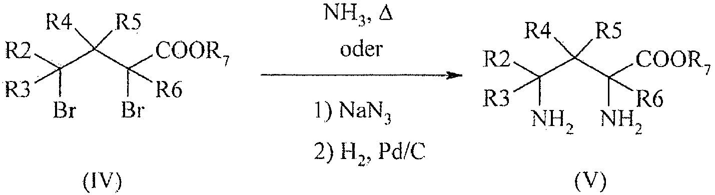

Von der Diaminoverbindung V wird ein Enantiomer gewonnen wie weiter unten beschrieben. Das erhaltene Enantiomer der Diaminoverbindung V wird schließlich, ggf. nach vorheriger Hydrolyse der Esterfunktion, zum gewünschten Produkt zyklisiert. Dies kann insbesondere durch Umsetzung mit einem geeigneten Orthoester R1-C(OR18)₃ erfolgen, wobei R1 = H, Alkyl, Cycloalkyl, Arylalkyl, Alkoxyalkyl, Alkylthioalkyl, Aryloxyalkyl oder Arylthioalkyl ist. Bei R18 handelt es sich um einen Alkylrest, insbesondere einen C₁-C₆-Alkylrest. Die Umsetzung erfolgt gemäß dem allgemeinen Schema, wie es von Koichi et al. beschrieben wurde (s.o.). Orthoester mit R18 = Me und R1 = Me oder Ph sind käuflich erhältlich.

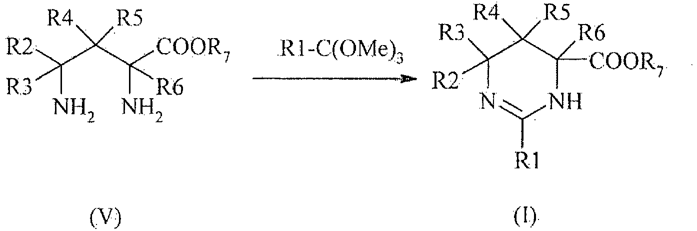

Anstelle der Verwendung eines Orthoesters ist auch eine Umsetzung mit einem Imidat oder Thioimidat denkbar. Die Reaktion mit käuflichem Methyl-acetimidat (R1= Me) bzw. Methyl-benzimidat (R1= Ph) ist im folgenden Schema dargestellt und allgemein durch J. Einsiedel et al., Bioorganic & Medicinal Chemistry Letters, 2003, 13, 851-854 beschrieben.

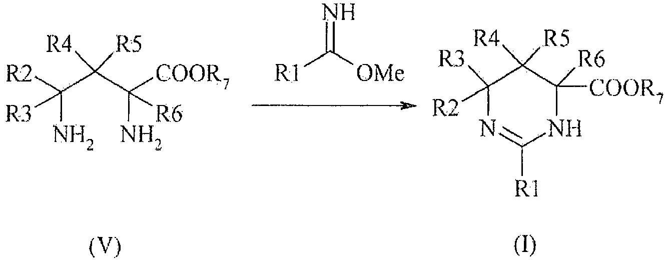

Durch Verwendung eines entsprechenden Orthoesters, Imidats oder Thioimidats kann der Substituent R1 des gewünschten Produkts eingestellt werden. Beispielsweise wird durch Verwendung von Orthoessigsäure-trimethylester (Trimethyl-orthoacetat, R1,18 = Methyl) auch ein Ectoinderivat erzeugt, bei dem R1 = Methyl ist.

Wie bereits erwähnt, kann vor oder nach Zyklisierung zum Amidin der allgemeinen Formel I auch eine Hydrolyse der Esterfunktion erfolgen, d. h. nach erfolgter Hydrolyse ist R7 = H, wobei anzumerken ist, dass Ectoin und seine Derivate, die über eine Carboxyfunktion verfügen, bei physiologischem pH als Zwitterion vorliegen. Selbstverständlich kann die Carboxyfunktion auch weiter derivatisiert werden, indem eine zusätzliche Veresterung durchgeführt wird. Es besteht somit eine große Bandbreite an Möglichkeiten zur Erzeugung unterschiedlicher Amidine der allgemeinen Formel I.

Eine weitere mögliche Derivatisierung der Carboxyfunktion besteht in der Umwandlung zum Amid. Das Produkt I, VI bzw. XI wird dazu gemäß gängiger Verfahren von einer Carbonsäure bzw. einem Carbonsäureester in ein Carbonsäureamid umgewandelt. Dies erfolgt beispielsweise durch direkte Umsetzung des Esters oder auch über den Umweg der Umsetzung des entsprechenden Säurechlorids bzw. -anhydrids mit Ammoniak/einem Amin. Es ergeben sich somit Verbindungen der Grundstruktur:

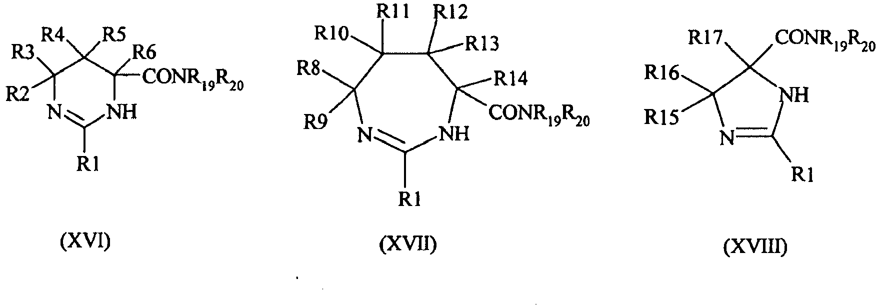

Dabei können R19 und R20 jeweils H oder Alkyl sein, wobei bevorzugt R20 = H ist. Insbesondere kann es sich bei R19 um eine langkettige Alkylkette handeln, beispielsweise um C₈-, C₉-, C₁₀-, C₁₁-, C₁₂-, C₁₃-, C₁₄-, C₁₅-, C₁₆-, C₁₇- oder C₁₈. Gleichzeitig handelt es sich bevorzugt bei R2 - R6, R8 - R14 bzw. R15 - R17 um H, während R1 = H oder Methyl ist, d. h. die Ringe tragen im Wesentlichen keine weiteren Substituenten. Derartige Amidine erscheinen aufgrund der erhöhten Lipophilie besonders vielversprechend.

Die verwendeten γ-Butyrolactone sind zum Teil käuflich erhältlich, beispielsweise R2 = Methyl, R3, R4, R5, R6 = H (CAS: 108-29-2) oder R2 = Phenyl, R3, R4, R5, R6 = H (CAS: 1008-76-0), R2, R3, R4, R5 = H, R6 = Methyl (CAS: 1679-47-6). Andere Lactone sind nicht käuflich, können aber gemäß in der Literatur beschriebener Verfahren hergestellt werden. So wird etwa in der WO 94/12487 A1 die Herstellung von α-Aryl-γ-butyrolactonen offenbart. Hierzu wird ein Anion des Malonats gemäß Formel 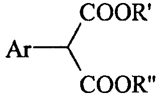 mit einer Ethylenverbindung der Formel Y-CH₂-CH₂-OZ umgesetzt, wobei Y eine Abgangsgruppe wie ein Halogen, Tosylat oder Mesylat und Z eine Schutzgruppe darstellt, wobei die Verbindung 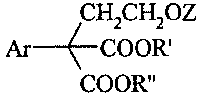 erhalten wird, die unter Hydrolyse das gewünschte Lacton ergibt.

Weitere Verfahren, die ebenfalls von Malonaten ausgehen, sind in B. Hoefgen et al., J. med. Chem., 2006, 49, 760-769 oder B. M. Nilsson et al., J. med. Chem. 1992, 35, 285-294 beschrieben. Butyrolactone mit einem Substituenten R6 ≠ H lassen sich beispielsweise gemäß dem folgenden Schema darstellen:

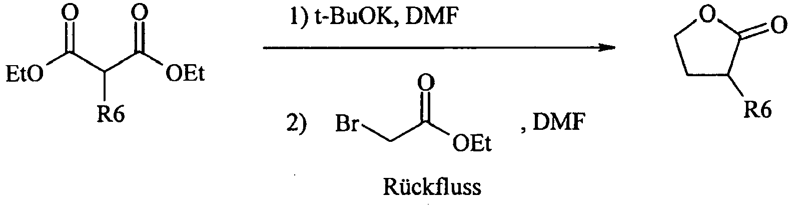

Die Herstellung von Butyrolactonen mit R5 ≠ H ist beispielsweise gemäß folgendem Schema möglich:

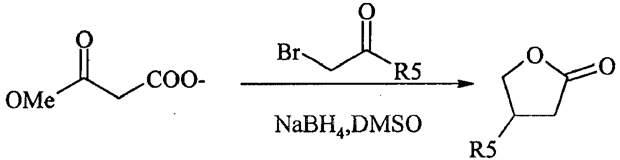

Weitere Beispiele lassen sich S. Schulz, Liebigs Ann. Chem. 1992, 8, 829-834 entnehmen.

In analoger Weise können nicht nur zyklische Amidine mit einem 6-Ring, sondern auch solche mit einem 5- oder 7-Ring hergestellt werden, deren Grundgerüst dem von DHMICA bzw. Homoectoin entspricht. Die Reaktionsreihenfolge stimmt mit der zuvor beschriebenen überein, allerdings wird nicht von einem γ-Butyrolacton, sondern von einem β- bzw. δ-Lacton ausgegangen. Dieses wird wiederum bromiert und anschließend verestert. Die Bromatome werden durch Aminogruppen substituiert, anschließend erfolgt der Ringschluss durch Reaktion mit einem Orthoester, Imidat oder Thioimidat, wobei dem Ringschluss wiederum die Gewinnung eines Enantiomers der Diaminoverbindung vorgeschaltet ist (s. u.). Die Reaktionsbedingungen sind weitgehend entsprechend, allerdings ist die Ausbeute beim Ringschluss zum 7-Ring erfahrungsgemäß schlechter, was im Wesentlichen auf die größere Ringspannung zurückzuführen ist. Man erhält auf diese Weise den 5-Ring XI bzw. den 7-Ring VI. Die Reaktionsschemata sind im folgenden dargestellt:

Synthese des Homoectoinderivates VI (R1, R7, R8, R9, R10, R11, R12, R13, R14 = H, Alkyl, Cycloalkyl, Arylalkyl, Alkoxyalkyl, Alkylthioalkyl, Aryloxyalkyl oder Arylthioalkyl):

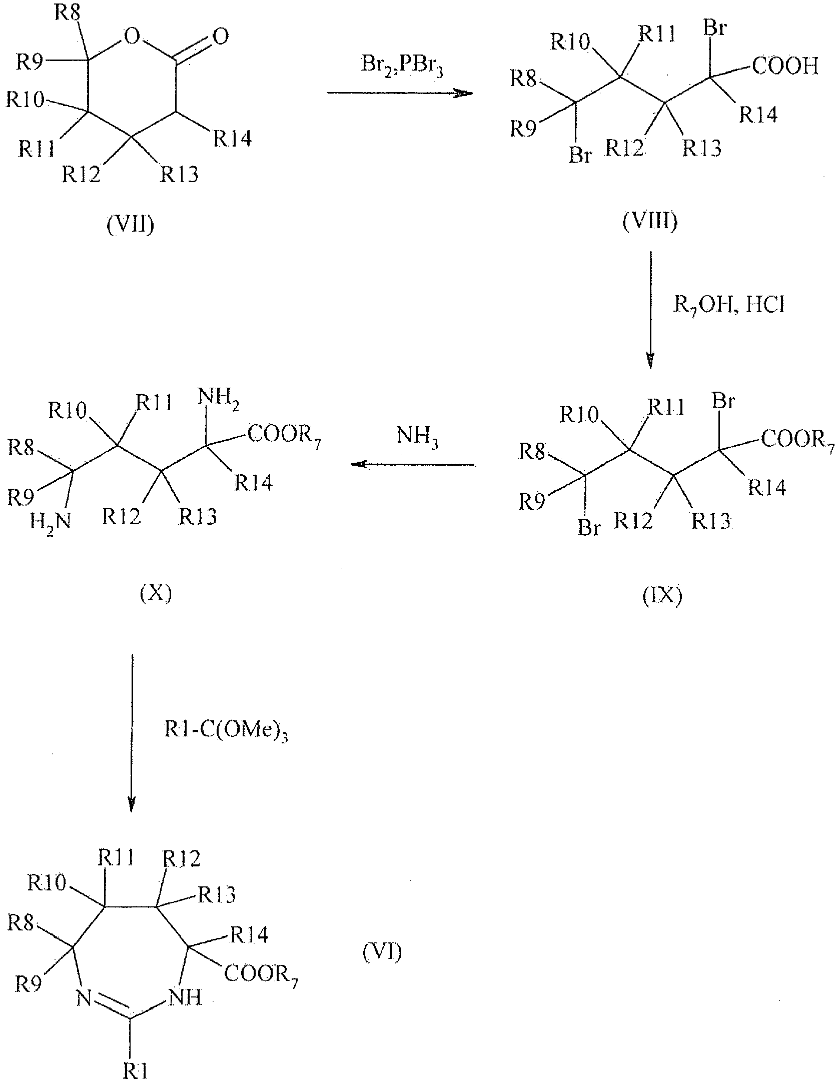

Synthese des DHMICA-Derivates XI (R1, R7, R15, R16, R17 = H, Alkyl, Cycloalkyl, Arylalkyl, Alkoxyalkyl, Alkylthioalkyl, Aryloxyalkyl oder Arylthioalkyl):

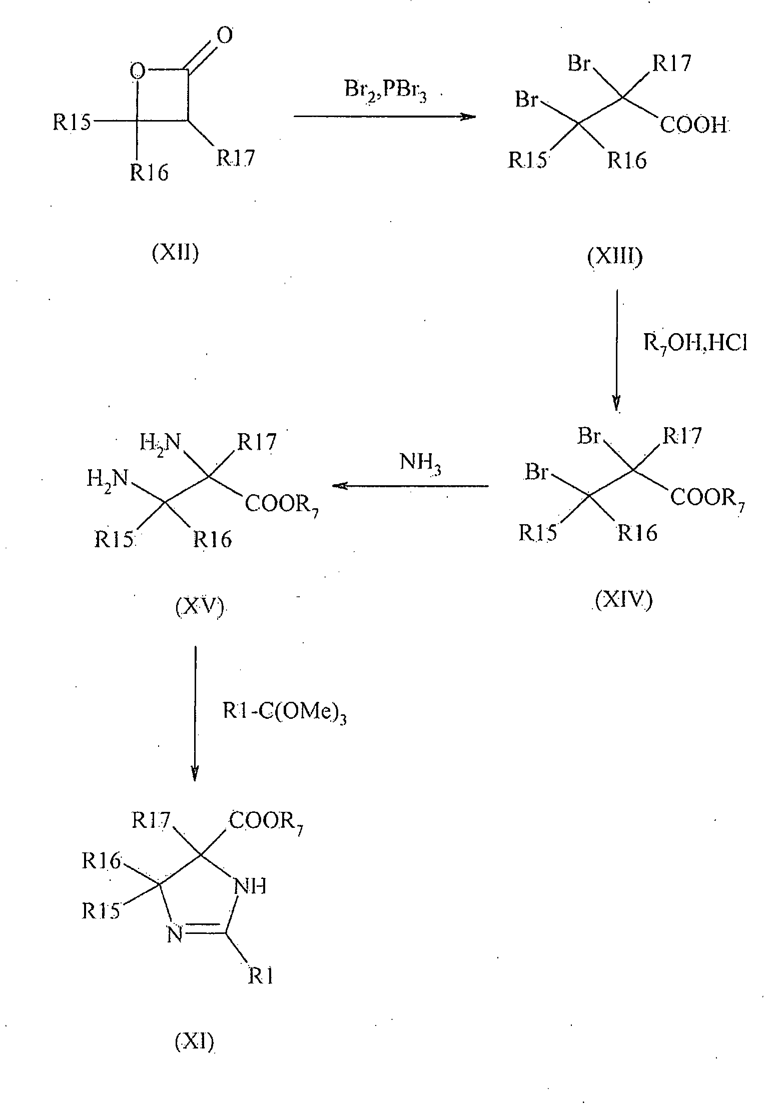

Gemäß dem erfindungsgemäßen Verfahren werden die zyklischen Amidine in enantiomerenreiner Form gewonnen. Es handelt sich somit um die Verbindungen

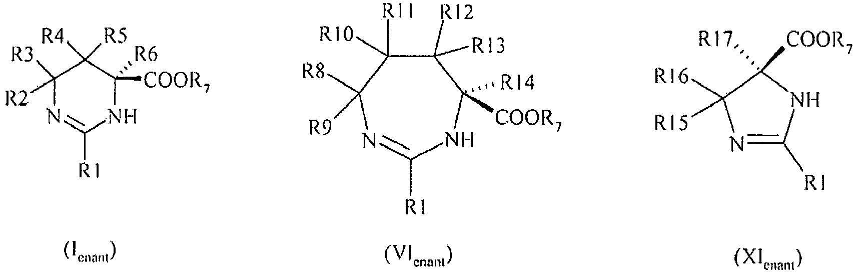

Dabei kann es sich insbesondere um das L-Enantiomer handeln, so dass beispielsweise das synthetisch hergestellte Ectoin dem natürlichen L-Ectoin entspricht.

Zur Herstellung eines zyklischen Amidins in enantiomerenreiner Form wird so vorgegangen, dass die Diaminoverbindung V, X bzw. XV in enantiomerenreiner Form gewonnen und anschließend wie oben beschrieben mit einem Orthoester, Imidat oder Thioimidat zum zyklischen Amidin der Formel I, VI oder XI umgesetzt wird. Das in der Diaminoverbindung befindliche Chiralitätszentrum bleibt dabei erhalten.

Die Diaminoverbindung wird zunächst an beiden Aminofunktionen acyliert, anschließend erfolgt eine stereoselektive Monodeacylierung mit Hilfe einer Aminoacylase. Lediglich ein Enantiomer der diacylierten Diaminoverbindung wird dabei an einer Aminofunktion deacyliert, während das andere Enantiomer an beiden Aminofunktionen acyliert bleibt. Hierfür geeignete Enzyme, insbesondere solche, die lediglich das L-Enantiomer einer Aminosäure hydrolysieren, sind hinlänglich bekannt. Von den beiden acylierten Aminogruppen wird dabei nur die Aminogruppe in α-Position zur Carboxylgruppe deacyliert. Es ergibt sich nach der Umsetzung mit der Aminoacylase somit ein Gemisch aus der Monoacylverbindung des einen und der Diacylverbindung des anderen Enantiomers.

Anschließend muss die Diacylverbindung des unerwünschten Enantiomers abgetrennt werden. Die verbleibende Monoacylverbindung des gewünschten Enantiomers wird sodann zur freien Diaminoverbindung V, X bzw. XV hydrolysiert. Es liegt nunmehr nur noch ein Enantiomer der Diaminoverbindung vor, welches im folgenden als Vₑₙₐₙₜ, Xₑₙₐₙₜ bzw. XVₑₙₐₙₜ bezeichnet wird. Die weitere Umsetzung zur gewünschten, enantiomerenreinen Verbindung Iₑₙₐₙₜ, VIₑₙₐₙₜ oder XIₑₙₐₙₜ erfolgt wie oben beschrieben. Sämtliche Ausführungen zur Herstellung der zyklischen Amidine gelten daher grundsätzlich auch für den Einsatz der Diaminoverbindung V, X oder XV als Enantiomer. Sofern vom Einsatz eines Enantiomers die Rede ist, kann die optische Reinheit auch unter 100 % liegen, wichtig ist jedoch, dass ein Enantiomer gegenüber dem anderen Enantiomer deutlich angereichert ist.

Bei der Acylierung der Diaminoverbindung V, X oder XV handelt es sich insbesondere um eine Acetylierung. Die Acylierung kann mit üblichen Acylierungsmitteln durchgeführt werden, beispielsweise mit Hilfe eines Carbonsäureanhydrids, eines Carbonsäurechlorids oder eines Carbonsäurebromids. Ebenso sind Imidazolide oder Carbonsäurethiolester wie 2-Pyridinthiolester verwendbar. Darüber hinaus sind im Stand der Technik weitere Verfahren bekannt, um die Acylgruppe einer Carbonsäure so zu aktivieren, dass eine Reaktion mit einer Aminofunktion zum Amid erfolgen kann, beispielsweise die Umsetzung mit Dicyclohexylcarbodiimid (DCC), 2-Chlorpyridinium- oder 3-Chloroxazolium-lonen etc. Besonders bevorzugt ist im Falle der Acetylierung der Einsatz von Acetanhydrid. Dies erfolgt auf übliche Weise im Alkalischen.

Die selektive Monodeacylierung erfolgt unter Einsatz einer Aminoacylase. Diese zur Familie der Hydrolasen gehörenden Enzyme sind in der Lage, die N-Acylgruppe an der Aminofunktion einer Aminosäure abzuspalten, und zwar selektiv nur bei einem Enantiomer. Zumeist erfolgt die Abspaltung nur beim L-Enantiomer der Aminosäure, bei dem es sich um das in der Natur hauptsächlich vorkommende Enantiomer handelt, während das D-Enantiomer nicht tangiert wird. Derartige Aminoacylasen werden daher auch als N-Acyl-L-aminosäureamidohydrolase bezeichnet. Besonders bevorzugt ist der Einsatz der Acylase I, insbesondere der Acylase I aus *Aspergillus.* Diese Acylase ist kommerziell verfügbar, beispielsweise bei der Fa. Fluka und wird auch in immobilisierter Form auf Eupergit vertrieben. Zusätzlich wird durch die Acylase I aus *Aspergillus* selektiv nur die Acylgruppe an der Aminofunktion in α-Stellung zur Carboxylgruppe abgespalten, während beispielsweise im Falle der *N^{α},N^{γ}*-Diacetyl-diaminobuttersäure die Acylgruppe an der γ-Aminofunktion nicht tangiert wird.

Es sind jedoch auch D-Aminoacylasen bekannt, die selektiv nur die N-Acylgruppe beim D-Enantiomer einer Aminosäure abspalten (vgl. z. B. C. S. Hsu et al., Protein Sci., 2002, 11, 2545 - 2550). Bei Einsatz einer solchen Acylase bleibt entsprechend das L-Enantiomer diacyliert und kann abgetrennt werden. Auf diese Weise stellt das erfindungsgemäße Verfahren einen Weg zu entsprechenden D-Enantiomeren der zu synthetisierenden zyklischen Amidine zur Verfügung, insbesondere auch zum D-Ectoin, D-Homoectoin und D-DHMICA.

Die Abtrennung des diacylierten, nicht gewünschten Enantiomers der Diaminoverbindung kann über einen Kationenaustauscher erfolgen. Dazu wird die Lösung nach Behandlung mit der Aminoacylase angesäuert und der Kationenaustauscher mit Wasser gewaschen. Anschließend kann im Alkalischen, beispielsweise mit einer NH₃-Lösung, das gewünschte Enantiomer eluiert werden.

Um anschließend auch die zweite Aminofunktion (in γ-, δ- oder β-Stellung) von der Acylgruppe zu befreien wird ein herkömmliches Verfahren der Amidhydrolyse zum Einsatz gebracht, insbesondere durch Anwendung einer Säure oder einer Base. Bevorzugt ist die Abspaltung der Acylgruppe im Sauren, beispielsweise durch Zugabe von HCl. Die gesamte Route zur Gewinnung eines Enantiomers einer Diaminoverbindung ist im folgenden anhand der 2,4-Diaminobuttersäure dargestellt. Diese ist käuflich erhältlich, kann aber auch über das erfindungsgemäße Verfahren gewonnen werden, wobei nach Herstellung der Diaminoverbindung eine Hydrolyse der Esterfunktion erfolgen muss.

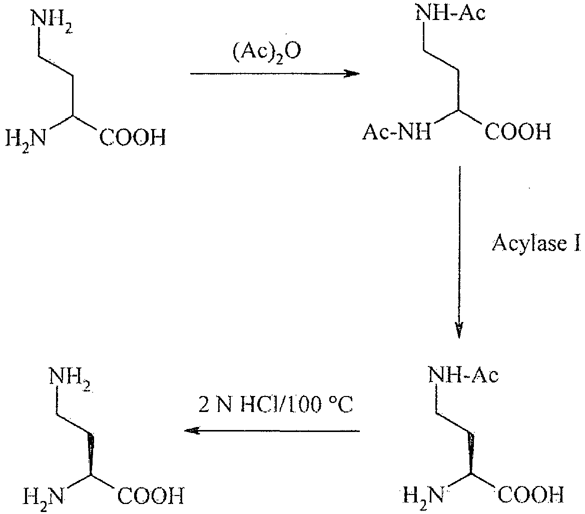

Bevorzugt handelt es sich bei den Resten R1 bis R17 um H, C₁- bis C₆-Alkyl oder Aryl. Zumeist ist dabei jeweils zumindest ein Substituent der Substituentenpaare R2-R3, R4-R5, R8-R9, R10-R11, R12-R13 und R15-R16 = H. Häufig wird eine Substitution lediglich an einer der Positionen R1 bis R17 vorliegen, während die jeweils anderen Reste ein H-Atom darstellen.

Neben dem erfindungsgemäßen Verfahren betrifft die Erfindung auch zyklische Amidine der allgemeinen Formeln 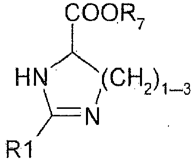 oder 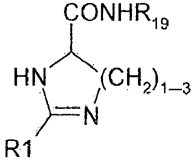 sowie hiervon abgeleitete Salze mit R1 = H oder Methyl, wobei R7 bzw. R19 ein C₈-, C₉-, C₁₀-, C₁₁-, C₁₂-, C₁₃-, C₁₄-, C₁₅-, C₁₆-, C₁₇- oder C₁₈-Alkylrest ist. Es ist zu erwarten, dass die entsprechenden zyklischen Amidine eine ähnliche Verwendbarkeit im medizinischen, kosmetischen oder biologischen Bereich aufweisen wie Ectoin oder Hydroxyectoin. Hierzu zählen die Verwendung als Wirkstoff in der Hautpflege und als Sonnenschutz, die Stabilisierung von Zellen, Proteinen, Nucleinsäuren und Biomembranen. Des Weiteren zu nennen sind der Schutz von Zellen, insbesondere Hautzellen vor Stressfaktoren wie Hitze, Kälte, UV-Strahlung und Trockenheit. Mit Hilfe des erfindungsgemäßen Verfahrens lassen sich nahezu beliebige anders funktionalisierte Ectoin-, Homoectoin- und DHMICA-Derivate herstellen, deren Eigenschaften je nach Bedarf angepasst werden können. So ist es beispielsweise möglich, in die Zielstrukturen I, VI und XI längere Alkylketten einzubauen, um die Lipophilie des Produktes zu erhöhen. Dies wiederum kann die Verwendbarkeit in Kosmetika verbessern. Ectoin selbst ist zwar hervorragend wasserlöslich, was sich schon durch seine Funktion als kompatibles Solut erklärt, bei der sich Ectoin in hoher Konzentration im Cytosol anreichert, die Löslichkeit in stärker lipophilen Umgebungen ist jedoch eingeschränkt. Dadurch, dass das erfindungsgemäße Verfahren hier eine große Bandbreite an Möglichkeiten zur Funktionalisierung eröffnet, kann die Löslichkeit des Ectoinderivats je nach Bedarf eingestellt werden.

Darüber hinaus betrifft die Erfindung auch zyklische Amidine in enantiomerenreiner Form, wie sie nach dem oben beschriebenen, modifizierten Verfahren hergestellt werden können. Das zyklische Amidin liegt somit bevorzugt als L-Enantiomer vor, denkbar ist jedoch auch das Vorliegen als D-Enantiomer, das hinsichtlich der Stereochemie vom natürlichen Ectoin abweicht. Die Enantiomerie bezieht sich auf das Chiralitätszentrum in α-Stellung zur Carboxylgruppe. Das L-Enantiomer ist dabei das Enantiomer, das in seiner Stereochemie dem natürlichen Ectoin ((S)-2-Methyl-1,4,5,6-tetrahydro-4-pyrimidincarbonsäure) entspricht, wobei zu berücksichtigen ist, dass Ectoin bei neutralem pH-Wert als Zwitterion vorliegt.

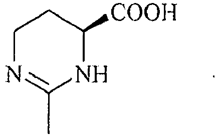

Die folgenden Beispiele dienen der weiteren Erläuterung der Erfindung:

### Beispiel 1: Synthese von Methyl-2-4-dibrombutyrat (2,4-Dibrombuttersäuremethylester

25 ml Brom wurden einer erhitzten Mischung von Phosphortribromid (0,83 ml) und γ-Butyrolacton (42 g, 0,49 mol) bei einer Temperatur von 100 bis 115 °C langsam zugetropft. Anschließend wurde die Mischung für weitere 30 min erhitzt. Die Mischung wurde im Eisbad gekühlt, 83 ml Methanol wurden vorsichtig hinzugefügt und gasförmiges HCl wurde eingeleitet. Die erhaltene Lösung wurde für 48 h auf 50 °C erhitzt. Die flüchtigen Komponenten wurden abgezogen, das verbleibende Öl wurde mit Diethylether verdünnt und zweimal mit 3 %iger wäßriger Natriumhydrogencarbonatlösung und NaCl-Lösung gewaschen. Die Waschlösungen wurden mit Diethylether rückextrahiert und die vereinigten organischen Phasen im Vakuum konzentriert. Die Destillation bei 0,2 Torr ergab das Produkt Methyl-2,4-dibrombutyrat in Form einer farblosen Flüssigkeit (100,4 g, 80 %).

### Beispiel 2: Synthese von 2,4-Diaminobuttersäuremethylester

Bei - 40 °C wurden in einem Kolben 150 ml Ammoniak einkondensiert und mit 150 ml wasserfreiem Diethylether verdünnt. Zu dieser Lösung wurden 100 g 2,4-Dibrombuttersäuremethylester (0,385 mol) in 100 ml wasserfreiem Diethylether langsam hinzugegeben. Bei - 40 °C wurde für 2 Stunden weitergerührt, anschließend wurde langsam auf Raumtemperatur erwärmt. Nach Rühren über Nacht wurde das Ammoniumbromid abfiltriert und das Lösungsmittel abgezogen. Es wurden 43,3 g (85 %) 2,4-Diaminobuttersäuremethylester erhalten.

### Beispiel 3: Synthese von 1,4,5,6-Tetrahydro-2-methyl-pyrimidin-4-carbonsäuremethylester

Eine Lösung von 2,4 g (0,018 mol) 2,4-Diaminobuttersäuremethylester und 3,77 g Orthoessigsäure-trimethylester in 50 ml getrocknetem Methanol wurden für 24 h im Rückfluß erhitzt. Die Lösung wurde bis zur Trockene eingeengt und das Produkt aus Methanol/Ethylacetat umkristallisiert. Es wurden 1,12 g (7,2 mmol, 40 %) 1,4,5,6-Tetrahydro-2-methyl-pyrimidin-4-carbonsäuremethylester erhalten.

### Beispiel 4: Synthese von L-2,4-Diaminobuttersäure

### 1) N^{α},N^{γ}-Diacetyl-D,L-diaminobuttersäure

Zu einer Lösung von D,L-2,4-Diaminobuttersäure (955 mg; 5 mmol) in 7,5 ml 2 N NaOH gab man unter Rühren und Eiskühlung in 5 Portionen Essigsäureanhydrid (1,42 ml; 15 mmol) und 7,5 ml 2 N NaOH. Mit zusätzlichen 1,5 ml 2 N NaOH wurde der pH-Wert auf 7,5 eingestellt. Nach 1 h im Eis wurde mit 2 ml 37 % HCl auf pH 3 angesäuert und die wässrige Phase 2 x mit je 50 ml n-Butanol/Ethylacetat (2:1) extrahiert. Nach Eindampfen der organischen Phase im Vakuum auf ein kleines Volumen wurde mit Methyl-*tert*-butylether gefällt. Es wurden 800 mg (79 %) *N^{α},N^{γ}*-Diacetyl-D,L-diaminobuttersäure als farbloser Feststoff erhalten. DC: R_{f} = 0,6 in Acetonitril/Wasser/Essigsäure (30:10:5); ESI-MS: m/z = 203,05 [M+H]⁺; Mᵣ = 202,21 ber. für C₈H₁₄N₂O₄

### 2) N^{γ}-Acetyl-L-diaminobuttersäure

*N^{α},N^{γ}*-Diacetyl-D,L-diaminobuttersäure (404 mg; 2 mmol) wurden in 20 ml Sörensen-Phosphat-Puffer (1/15 molar) bei pH 7,6 mit 28 mg Acylase I *Aspergillus* (Fluka, 0,72 U7mg Enzym; entspricht 10 U/1 mmol Substrat) versetzt und 20 h im Wasserbad bei 40 °C inkubiert. Das Enzym wurde mittels Membranfilter Amicon Ultra-4 (Ausschlussgrenze 10 kD) abgetrennt und die Lösung mit 1 N HCl auf pH 2,5 eingestellt.

Nach Aufgabe auf 50 ml Kationenaustauscher Dowex 50Wx8 (50 - 100 mesh) wurde mit Wasser bis pH 5 gewaschen (Abtrennung der Diacetyl-Verbindung) und anschließend das gewünschte Produkt mit 1 N Ammoniak eluiert. Das Eluat wurde im Vakuum eingedampft, wobei sofort farblose Kristalle von *N^{γ}*-Acetyl-L-diaminobuttersäure entstanden. Ausbeute: 140 mg (88 % der erwarteten 50 %-igen Ausbeute); DC: R_{f} = 0,3 in Acetonitril/Wasser/Essigsäure (30:10:5); ESI-MS: m/z = 161,10 [M+H]⁺; Mᵣ = 160,17 ber. für C₆H₁₂N₂O₃

### 3) L-Diaminobuttersäure

*N^{γ}*-Acetyl-L-diaminobuttersäure (140 mg; 0,87 mmol) wurden in 10 ml 2 N HCl unter Rückfluss gerührt. Nach Eindampfen i. Vakuum wurde der kristalline Rückstand in möglichst wenig Wasser gelöst und mit 4 ml heißem Ethanol abs. gefällt. Die L-Diaminobuttersäure fiel als Monohydrochlorid farblos an. Ausbeute: 125 mg (93 %); DC: R_{f} = 0,1 in Acetonitril/Wasser/Essigsäure (30:10:5); ESI-MS: m/z = 119,08 [M+H]⁺; Mᵣ = 118,14 ber. für C₄H₁₀N₂O₂; Mᵣ = 154,60 ber. für C₄H₁₀N₂O₂ x HCl; [α]²⁰_{D} = + 24,4° (c = 1,2 in 6 N HCl); Lit. (Beilstein, 4/IV, 2613): [α]²¹_{D} = + 23,8° (c = 1 in 6 N HCl); Fluka-Katalog: [α]²⁰_{D} = 24 ± 2° (c =1 in 6 N HCl). Die Enantiomerenreinheit der Aminosäure wurde mittels Derivatisierung mit Marlfey's Reagenz (J. G. Adamson et al., Anal. Biochem., 1992, 202, 210 - 214) und anschließender HPLC bestätigt. Bedingungen: Säule ET 125/4 Nucleosil 100-5 C8 (Macherey und Nagel, Düren); linear Gradient von Eluent A (1 % Trifluoressigsäure (TFA) in H₂0) zu Eluent B (0,8 % TFA in Acetonitril); Rₜ = 15,6 min.

## Patentansprüche

1. Verfahren zur Herstellung von
zyklischen Amidinen der allgemeinen Formel I 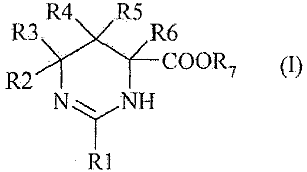 wobei R1, R2, R3, R4, R5, R6, R7 = H, Alkyl, Cycloalkyl, Arylalkyl, Alkoxyalkyl, Alkylthioalkyl, Aryloxyalkyl oder Arylthioalkyl ist
mit folgenden Schritten:
- Bromierung eines Lactons der allgemeinen Formel II 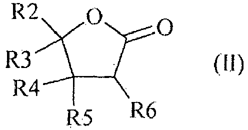 zur Dibromverbindung der allgemeinen Formel III 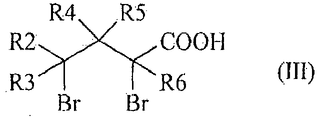
- Veresterung der Dibromverbindung III zur Verbindung IV 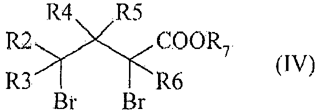
- Aminierung der Dibromverbindung IV zur Diaminoverbindung V 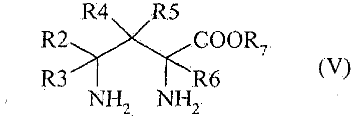
- Gewinnung eines Enantiomers der Verbindung V durch die Unterschritte
▪ Acylierung der Verbindung V an beiden Aminofunktionen zur Diacylverbindung
▪ stereoselektive Monodeacylierung eines Enantiomers der Diacylverbindung in α-Position durch Einsatz einer Aminoacylase zur Monoacylverbindung
▪ Abtrennung der nicht deacylierten Diacylverbindung
▪ Hydrolyse der Monoacylverbindung zum Enantiomer der Verbindung V
- Umsetzung des Enantiomers der Verbindung V mit einem Orthoester der allgemeinen Formel R1-C(OR18)₃, wobei R1 die vorstehend angegebene Bedeutung hat und R18 = Alkyl, insbesondere C₁ - C₆-Alkyl, ist, oder einem Imidat der allgemeinen Formel 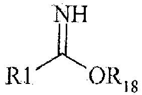 wobei R1, R18 die vorstehend angegebene Bedeutung haben, oder einem Thioimidat der allgemeinen Formel 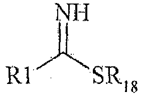
wobei R1, R18 die vorstehend angegebene Bedeutung haben, zum zyklischen Amidin der allgemeinen Formel I.

2. Verfahren zur Herstellung von
zyklischen Amidinen der allgemeinen Formel VI 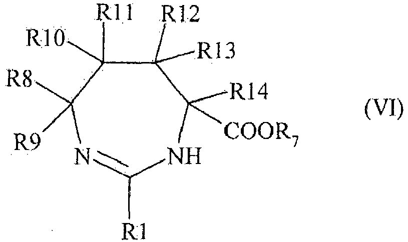 wobei R1, R7, R8, R9, R10, R11, R12, R13, R14 = H, Alkyl, Cycloalkyl, Arylalkyl, Alkoxyalkyl, Alkylthioalkyl, Aryloxyalkyl oder Arylthioalkyl ist
mit folgenden Schritten:
- Bromierung eines Lactons der allgemeinen Formel VII 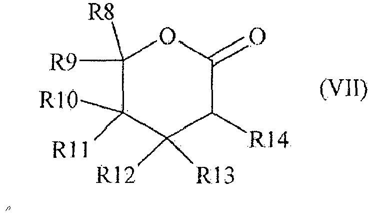 zur Dibromverbindung der allgemeinen Formel VIII 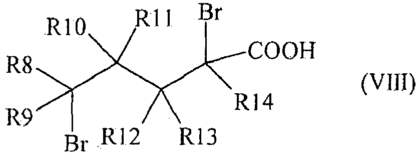
- Veresterung der Dibromverbindung VIII zur Verbindung IX 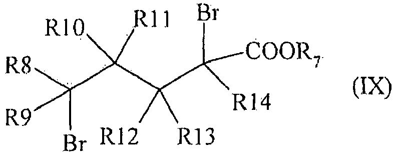
- Aminierung der Dibromverbindung IX zur Diaminoverbindung X 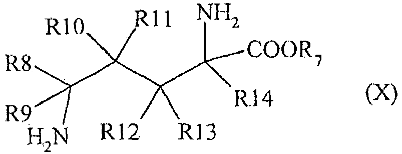
- Gewinnung eines Enantiomers der Verbindung X durch die Unterschritte
▪ Acylierung der Verbindung X an beiden Aminofunktionen zur Diacylverbind ung
▪ stereoselektive Monodeacylierung eines Enantiomers der Diacylverbindung in α-Position durch Einsatz einer Aminoacylase zur Monoacylverbindung
▪ Abtrennung der nicht deacylierten Diacylverbindung
▪ Hydrolyse der Monoacylverbindung zum Enantiomer der Verbindung X
- Umsetzung des Enantiomers der Verbindung X mit einem Orthoester der allgemeinen Formel R1-C(OR18)₃, wobei R1 die vorstehend angegebene Bedeutung hat und R18 = Alkyl, insbesondere C₁ - C₆-Alkyl, ist, oder einem Imidat der allgemeinen Formel 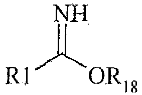 wobei R1, R18 die vorstehend angegebene Bedeutung haben, oder einem Thioimidat der allgemeinen Formel 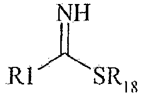
wobei R1, R18 die vorstehend angegebene Bedeutung haben, zum zyklischen Amidin der allgemeinen Formel VI.

3. Verfahren zur Herstellung von
zyklischen Amidinen der allgemeinen Formel XI 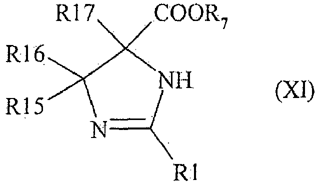 wobei R1, R7, R15, R16, R17 = H, Alkyl, Cycloalkyl, Arylalkyl, Alkoxyalkyl, Alkylthioalkyl, Aryloxyalkyl oder Arylthioalkyl ist
mit folgenden Schritten:
- Bromierung eines Lactons der allgemeinen Formel XII 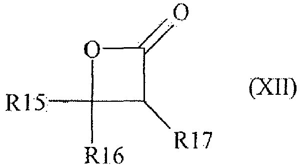 zur Dibromverbindung der allgemeinen Formel XIII 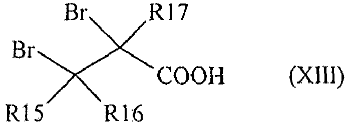
- Veresterung der Dibromverbindung XIII zur Verbindung XIV 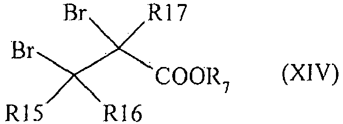
- Aminierung der Dibromverbindung XIV zur Diaminoverbindung XV 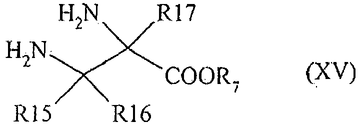
- Gewinnung eines Enantiomers der Verbindung XV durch die Unterschritte
▪ Acylierung der Verbindung XV an beiden Aminofunktionen zur Diacylverbindung
▪ stereoselektive Monodeacylierung eines Enantiomers der Diacylverbindung in α-Position durch Einsatz einer Aminoacylase zur Monoacylverbindung
▪ Abtrennung der nicht deacylierten Diacylverbindung
▪ Hydrolyse der Monoacylverbindung zum Enantiomer der Verbindung XV
- Umsetzung des Enantiomers der Verbindung XV mit einem Orthoester der allgemeinen Formel R1-C(OR18)₃, wobei R1 die vorstehend angegebene Bedeutung hat und R18 = Alkyl, insbesondere C₁ - C₆-Alkyl, ist, oder einem Imidat der allgemeinen Formel 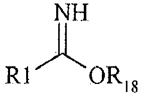 wobei R1, R18 die vorstehend angegebene Bedeutung haben, oder einem Thioimidat der allgemeinen Formel 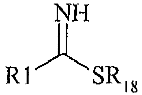
wobei R1, R18 die vorstehend angegebene Bedeutung haben, zum zyklischen Amidin der allgemeinen Formel XI.

4. Verfahren nach einem der Ansprüche 1 bis 3, **dadurch gekennzeichnet, dass** die Esterfunktion der Verbindungen V, X oder XV vor dem Schritt der Umsetzung mit dem Orthoester, Imidat oder Thioimidat hydrolysiert wird.

5. Verfahren nach einem der Ansprüche 1 bis 4, **dadurch gekennzeichnet, dass** die Aminierung der Dibromverbindung IV, IX oder XIV durch Umsetzung mit einem Azid und anschließende Hydrierung oder durch Umsetzung mit NH₃ erfolgt.

6. Verfahren nach einem der Ansprüche 1 bis 5, **dadurch gekennzeichnet, dass** R1-17 = H, C₁- bis C₆-Alkyl oder Aryl ist.

7. Verfahren nach einem der Ansprüche 1 bis 6, **dadurch gekennzeichnet, dass** jeweils mindestens ein Substituent der Substituentenpaare R2-R3, R4-R5, R8-R9, R10-R11, R12-R13 und R15-R16 = H ist.

8. Verfahren nach einem der Ansprüche 1 bis 7, **dadurch gekennzeichnet, dass** die Carbonsäure- oder Carbonsäureesterfunktion COOR7 zu einer Carbonsäureamidfunktion CONR19R20 umgewandelt wird, wobei R19 und R20 = H oder Alkyl sind.

9. Verfahren nach einem der Ansprüche 1 bis 8, **dadurch gekennzeichnet, dass** das Enantiomer das L-Enantiomer ist.

10. Verfahren nach einem der Ansprüche 1 bis 9, **dadurch gekennzeichnet, dass** die Acylgruppe eine Acetylgruppe ist.

11. Verfahren nach einem der Ansprüche 1 bis 10, **dadurch gekennzeichnet, dass** die Aminoacylase Acylase I aus *Aspergillus* ist.

12. Zyklisches Amidin der Formel 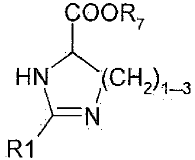 sowie dessen Salze
mit R1 = H oder Methyl, wobei R7 ein C₈-, C₉-, C₁₀-, C₁₁-, C₁₂-, C₁₃-, C₁₄-, C₁₅-, C₁₆-, C₁₇- oder C₁₈-Alkylrest ist.

13. Zyklisches Amidin nach Anspruch 12, **dadurch gekennzeichnet, dass** das Amidin als L- oder D-Enantiomer vorliegt.

14. Zyklisches Amidin der Formel 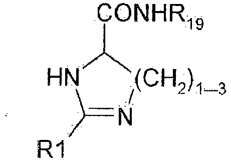 sowie dessen Salze
mit R1 = H oder Methyl, wobei R19 = C₈-, C₉-, C₁₀-, C₁₁-, C₁₂-, C₁₃-, C₁₄-, C₁₅-, C₁₆-, C₁₇- oder C₁₈-Alkylrest ist.

## Claims

1. Method for preparation of
cyclic amidines of the general formula I 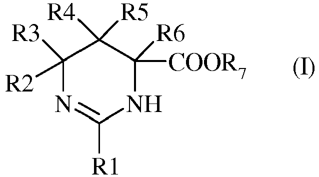 with R1, R2, R3, R4, R5, R6, R7 = H, alkyl, cycloalkyl, arylalkyl, alkoxyalkyl, alkylthioalkyl, aryloxyalkyl or arylthioalkyl
comprising the following steps:
- Bromination of a lactone of the general formula II 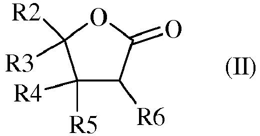 to a dibromine compound of the general formula III 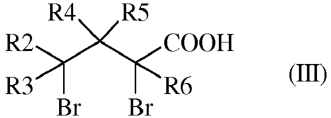
- Esterification of the dibromine compound III to a compound IV 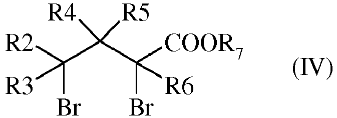
- Amination of the dibromine compound IV to a diamino compound V 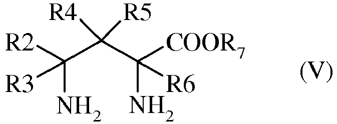
- Obtaining an enantiomer of the compound V by the sub-steps
• Acylation of the compound V at both amino functions to give the diacyl compound
• Stereo-selective monodeacylation of an enantiomer of the diacyl compound in α-position by use of an aminoacylase to give the monoacyl compound
• Separation of the non-deacylated diacyl compound
• Hydrolysis of the monoacyl compound to the enantiomer of compound V
- Reaction of the enantiomer of compound V with an ortho-ester of the general formula R1-C(OR18)₃, with R1 having the meaning outlined hereinabove and R18 = alkyl, in particular C₁ - C₆-alkyl, or an imidate of the general formula 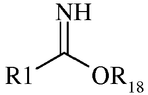
with R1, R18 having the meaning as outlined hereinabove, or a thioimidate of the general formula 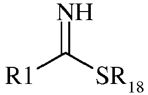 with R1, R18 having the meaning as outlined hereinabove, to obtain a cyclic amidine of the general formula I.

2. Method for preparation of
cyclic amidines of the general formula VI 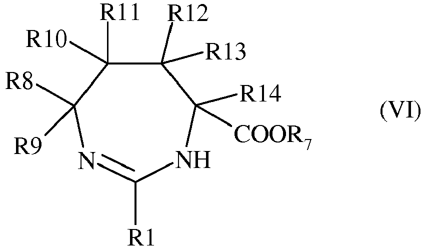 with R1, R7, R8, R9, R10, R11, R12, R13, R14 = H, alkyl, cycloalkyl,, arylalkyl, alkoxyalkyl, alkylthioalkyl, aryloxyalkyl or arylthioalkyl
comprising the following steps:
- Bromination of a lactone of the general formula VII 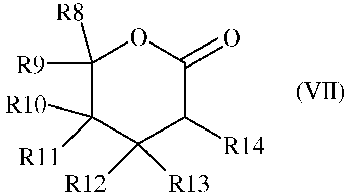 to a dibromine compound of the general formula VIII 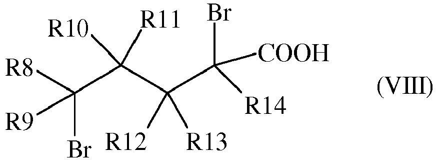
- Esterification of the dibromine compound VIII to a compound IX 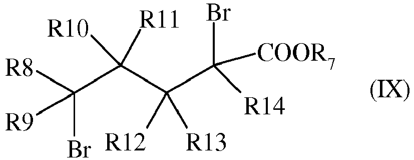
- Amination of the dibromine compound IX to a diamino compound X 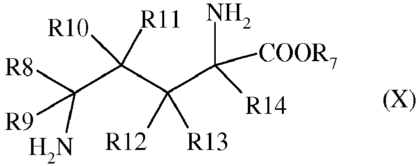
- Obtaining an enantiomer of the compound X by the sub-steps
• Acylation of the compound X at both amino functions to give the diacyl compound
• Stereo-selective monodeacylation of an enantiomer of the diacyl compound in α-position by use of an aminoacylase to give the monoacyl compound
• Separation of the non-deacylated diacyl compound
• Hydrolysis of the monoacyl compound to the enantiomer of compound X
- Reaction of the enantiomer of compound X with an ortho-ester of the general formula R1-C(OR18)₃, with R1 having the meaning outlined hereinabove and R18 = alkyl, in particular C₁ - C₆-alkyl, or an imidate of the general formula 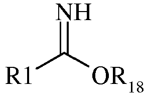 with R1, R18 having the meaning as outlined hereinabove, or a thioimidate of the general formula 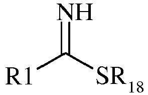
with R1, R18 having the meaning as outlined hereinabove, to obtain a cyclic amidine of the general formula VI.

3. Method for preparation of
cyclic amidines of the general formula XI 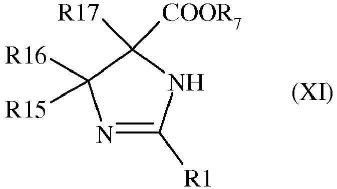 with R1, R7, R15, R16, R17 = H, alkyl, cycloalkyl, arylalkyl, alkoxyalkyl, alkylthioalkyl, aryloxyalkyl or arylthioalkyl
comprising the following steps:
- Bromination of a lactone of the general formula XII 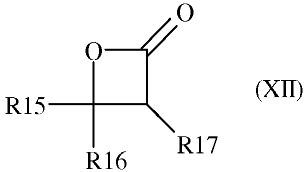 to a dibromine compound of the general formula XIII 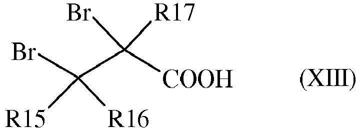
- Esterification of the dibromine compound XIII to a compound XIV 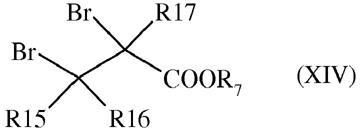
- Amination of the dibromine compound XIV to a diamino compound XV 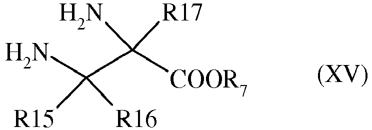
- Obtaining an enantiomer of the compound XV by the sub-steps
• Acylation of the compound XV at both amino functions to give the diacyl compound
• Stereo-selective monodeacylation of an enantiomer of the diacyl compound in α-position by use of an aminoacylase to give the monoacyl compound
• Separation of the non-deacylated diacyl compound
• Hydrolysis of the monoacyl compound to the enantiomer of compound XV
- Reaction of the enantiomer of compound XV with an ortho-ester of the general formula R1-C(OR18)₃, with R1 having the meaning outlined hereinabove and R18 = alkyl, in particular C₁ - C₆-alkyl, or an imidate of the general formula 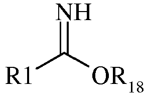 with R1, R18 having the meaning as outlined hereinabove, or a thioimidate of the general formula 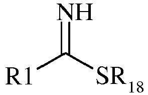
with R1, R18 having the meaning as outlined hereinabove, to obtain a cyclic amidine of the general formula XI.

4. Method according to any of claims 1 to 3, **characterized in that** the ester function of the compounds V, X or XV is hydrolyzed prior to the step of the reaction with the orthoester, imidate or thioimidate.

5. Method according to any of claims 1 to 4, **characterized in that** the amination of the dibromine compound IV, IX or XIV is realized by reaction with an azide and a subsequent hydrogenation or by reaction with NH₃.

6. Method according to any of claims 1 to 5, **characterized in that** R1-17 is = H, C₁- to C₆-alkyl or aryl.

7. Method according to any of claims 1 to 6, **characterized in that** in each case at least one substituent of the substituent couples R2-R3, R4-R5, R8-R9, R10-R11, R12-R13 and R15-R16 is = H.

8. Method according to any of claims 1 to 7, **characterized in that** the carboxylic acid or carboxylic acid ester function COOR7 is converted to a carboxylic acid amide function CONR19R20, with R19 and R20 being = H or alkyl.

9. Method according to any of claims 1 to 8, **characterized in that** the enantiomer is the L-enantiomer.

10. Method according to any of claims 1 to 9, **characterized in that** the acyl group is an acetyl group.

11. Method according to any of claims 1 to 10, **characterized in that** the amino acylase is an acylase I from *aspergillus.*

12. Cyclic amidine of the formula I 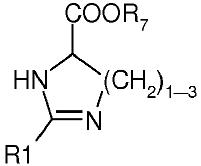 as well as its salts
with R1 = H or methyl, with R7 being C₈-, C₉-, C₁₀-, C₁₁-, C₁₂-, C₁₃-, C₁₄-, C₁₅-, C₁₆-, C₁₇- or C₁₈-alkyl moiety.

13. Cyclic amidine according to claim 12, **characterized in that** the amidine is present as an L- or D-enantiomer.

14. Cyclic amidine of the formula 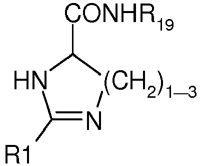 as well as its salts
with R1 = H or methyl, with R19 being C₈-, C₉-, C₁₀-, C₁₁-, C₁₂-, C₁₃-, C₁₄-, C₁₅-, C₁₆-, C₁₇- or C₁₈-alkyl moiety.

## Revendications

1. Procédé pour la préparation d'amidines cycliques de formule générale I 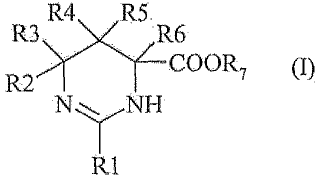 dans laquelle R1, R2, R3, R4, R5, R6, R7 = H, alkyle, cycloalkyle, arylalkyle, alcoxyalkyle, alkylthioalkyle, aryloxyalkyle ou arylthioalkyle, présentant les étapes suivantes :
- bromuration d'une lactone de formule générale II 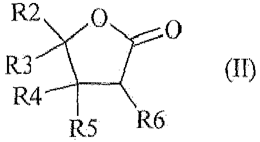 en composé dibromé de formule générale III 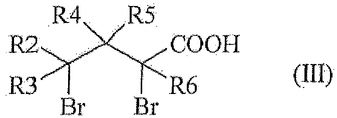
- estérification du composé dibromé III en composé IV 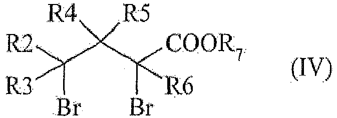
- amination du composé dibromé IV en composé diamino V 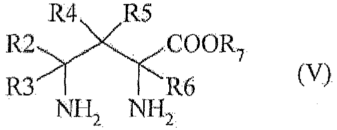
- obtention d'un énantiomère du composé V par les sous-étapes
- acylation du composé V sur les deux fonctions amino en composé diacyle
- monodésacylation stéréosélective d'un énantiomère du composé diacyle en position α par l'utilisation d'une aminoacylase en composé monoacyle
- séparation du composé diacyle non désacylé
- hydrolyse du composé monoacyle en énantiomère du composé V
- transformation de l'énantiomère du composé V avec un orthoester de formule générale R1-C(OR18)₃, dans laquelle R1 présente la signification indiquée ci-dessus et R18 = alkyle, en particulier C₁-C₆-alkyle, ou avec un imidate de formule générale 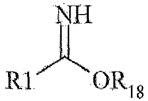 dans laquelle R1, R18 présentent la signification indiquée ci-dessus ou avec un thio-imidate de formule générale 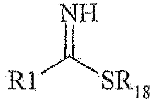
dans laquelle R1, R18 présentent la signification indiquée ci-dessus en amidine cyclique de formule générale I.

2. Procédé pour la préparation d'amidines cycliques de formule générale VI 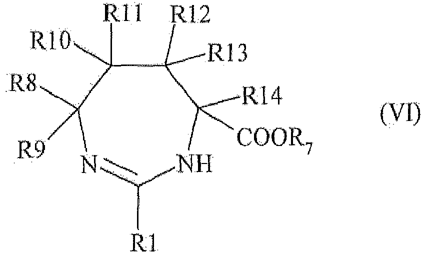 dans laquelle R1, R7, R8, R9, R10, R11, R12, R13, R14 = H, alkyle, cycloalkyle, arylalkyle, alcoxyalkyle, alkylthioalkyle, aryloxyalkyle ou arylthioalkyle, présentant les étapes suivantes :
- bromuration d'une lactone de formule générale VII 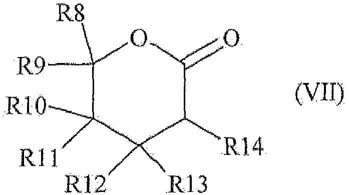
- en composé dibromé de formule générale VIII 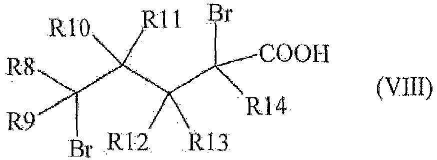
- estérification du composé dibromé VIII en composé IX 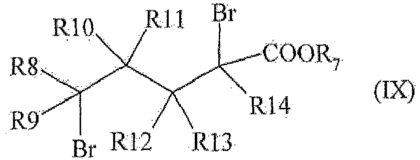
- amination du composé dibromé I en composé diamino X 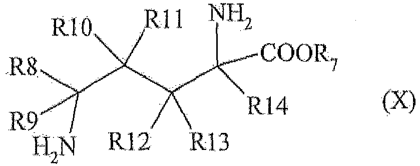
- obtention d'un énantiomère du composé X par les sous-étapes
- acylation du composé X sur les deux fonctions amino en composé diacyle
- monodésacylation stéréosélective d'un énantiomère du composé diacyle en position α par utilisation d'une aminoacylase en composé monoacyle
- séparation du composé diacyle non désacylé
- hydrolyse du composé monoacyle en énantiomère du composé X
- transformation de l'énantiomère du composé X avec un orthoester de formule générale R1-C(OR18)₃, dans laquelle R1 présente la signification indiquée ci-dessus et R18 = alkyle, en particulier C₁-C₆-alkyle, ou avec un imidate de formule générale 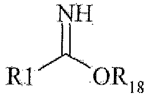 dans laquelle R1, R18 présentent la signification indiquée ci-dessus ou avec un thio-imidate de formule générale 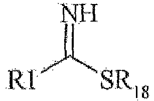
dans laquelle R1, R18 présentent la signification indiquée ci-dessus en amidine cyclique de formule générale VI.

3. Procédé pour la préparation d'amidines cycliques de formule générale XI 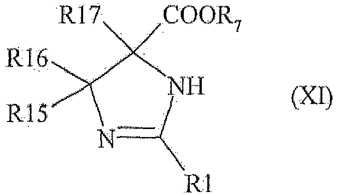 dans laquelle R1, R7, R15, R16, R17 = H, alkyle, cycloalkyle, arylalkyle, alcoxyalkyle, alkylthioalkyle, aryloxyalkyle ou arylthioalkyle, présentant les étapes suivantes :
- bromuration d'une lactone de formule générale XII 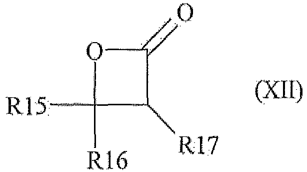 en composé dibromé de formule générale XIII 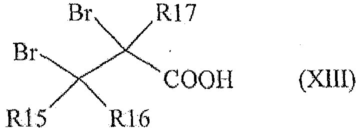
- estérification du composé dibromé XIII en composé XIV 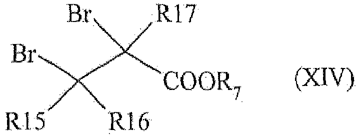
- amination du composé dibromé XIV en composé diamino XV 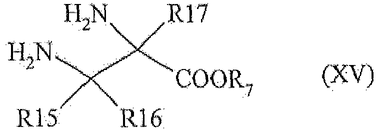
- obtention d'un énantiomère du composé XV par les sous-étapes
- acylation du composé XV sur les deux fonctions amino en composé diacyle
- monodésacylation stéréosélective d'un énantiomère du composé diacyle en position α par utilisation d'une aminoacylase en composé monoacyle
- séparation du composé diacyle non désacylé
- hydrolyse du composé monoacyle en énantiomère du composé XV
- transformation de l'énantiomère du composé XV avec un orthoester de formule générale R1-C(OR18)₃, dans laquelle R1 présente la signification indiquée ci-dessus et R18 = alkyle, en particulier C₁-C₆-alkyle, ou avec un imidate de formule générale 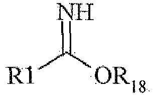 dans laquelle R1, R18 présentent la signification indiquée ci-dessus ou avec un thio-imidate de formule générale 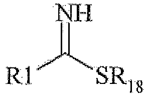
dans laquelle R1, R18 présentent la signification indiquée ci-dessus en amidine cyclique de formule générale XI.

4. Procédé selon l'une quelconque des revendications 1 à 3, **caractérisé en ce que** la fonction ester des composés V, X ou XV est hydrolysée avant l'étape de la transformation avec l'orthoester, l'imidate ou le thio-imidate.

5. Procédé selon l'une quelconque des revendications 1 à 4, **caractérisé en ce que** l'amination du composé dibromé IV, IX ou XIV est effectuée par transformation avec un azide et hydrogénation consécutive ou par transformation avec NH₃.

6. Procédé selon l'une quelconque des revendications 1 à 5, **caractérisé en ce que** R1-17 = H, C₁-C₆-alkyle ou aryle.

7. Procédé selon l'une quelconque des revendications 1 à 6, **caractérisé en ce qu'**à chaque fois au moins un substituant des paires de substituants R2-R3, R4-R5, R8-R9, R10-R11, R12-R13 et R15-R16 = H.

8. Procédé selon l'une quelconque des revendications 1 à 7, **caractérisé en ce que** la fonction acide carboxylique ou ester d'acide carboxylique COOR7 est transformée en une fonction amide d'acide carboxylique CONR19R20 dans laquelle R19 et R20 = H ou alkyle.

9. Procédé selon l'une quelconque des revendications 1 à 8, **caractérisé en ce que** l'énantiomère est l'énantiomère L.

10. Procédé selon l'une quelconque des revendications 1 à 9, **caractérisé en ce que** le groupe acyle est un groupe acétyle.

11. Procédé selon l'une quelconque des revendications 1 à 10, **caractérisé en ce que** l'aminoacylase est l'acylase I d'Aspergillus.

12. Amidine cyclique de formule 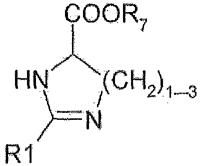 ainsi que ses sels, dans laquelle R1 = H ou méthyle, R7 représentant un radical alkyle en C₈, C₉, C₁₀, C₁₁, C₁₂, C₁₃, Cₗ₄, C₁₅, C₁₆, C₁₇ ou C₁₈.

13. Amidine cyclique selon la revendication 12, **caractérisée en ce que** l'amidine se trouve sous forme d'énantiomère L ou D.

14. Amidine cyclique de formule 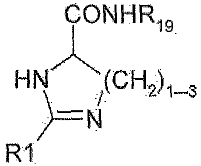 ainsi que ses sels, dans laquelle R1 = H ou méthyle, R19 = un radical alkyle en C₈, C₉, C₁₀, C₁₁, C₁₂, C₁₃, C₁₄, C₁₅, C₁₆, C₁₇ ou C₁₈.
